Europäisches Patentamt

**European Patent Office**

Office européen des brevets

(19)

(11) Numéro de publication: **0 029 752**
**B1**

(12) **FASCICULE DE BREVET EUROPEEN**

(45) Date de publication du fascicule du brevet: **27.04.83**

(51) Int. Cl.³: **A 61 B 17/18**

(21) Numéro de dépôt: **80400739.1**

(22) Date de dépôt: **27.05.80**

(54) **Implant utilisant un clou élastique pour la réduction de fracture trochantérienne.**

(30) Priorité: **21.11.79 FR 7929018**

(43) Date de publication de la demande:
**03.06.81 Bulletin 81/22**

(45) Mention de la délivrance du brevet:
**27.04.83 Bulletin 83/17**

(84) Etats contractants désignés:
**BE DE GB IT LU NL**

(56) Documents cités:
**CH - A - 576 249**
**FR - A - 2 210 908**
**FR - A - 2 237 609**
**FR - A - 2 254 298**

(73) Titulaire: **Landanger, Louis**
**10, Rue de Dijon**
**F-52000 Chaumont (FR)**

(72) Inventeur: **Kempf, Yvan**
**Centre de Traumatologie Illkirsch Graffenstaden**
**F-67000 Strasbourg (FR)**
Inventeur: **Bittar, Suleimann**
**Centre de traumatologie Illkirsch Graffenstaden**
**F-67000 Strasbourg (FR)**

(74) Mandataire: **Martinet, René et al,**
**Cabinet Martinet 62, rue des Mathurins**
**F-75008 Paris (FR)**

Courier Press, Leamington Spa, England.

Implant utilisant un clou élastique pour la réduction de fracture trochantérienne

L'invention est du domaine des implants chirurgicaux. Elle concerne un clou élastique perfectionné pour le traitement des fractures trochantériennes.

De la demande de brevet FR—A— 2 237 609 on connait, pour la réduction des fractures trochantériennes chez les personnes agées en particulier, la technique de l'enclouage fasciculé de ENDER selon laquelle un faisceau de clous est passé dans le canal médullaire par un orifice d'introduction pratiqué dans l'apophyse inférieure du fémur.

Cette technique entraîne cependant souvent de nombreuses complications, essentiellement la descente des clous et une rotation externe excessive, avec leurs conséquences néfastes: douleurs au niveau du genou, perforation cutanée, démontage de la fracture, cals vicieux et reprises opératoires.

La première tentative des chirurgiens, devant le problème de descente des clous, a été de pratiquer un meilleur remplissage du canal médullaire. Ils se sont afforcés à mettre en place non seulement quatre clous mais cinq, six et parfois sept clous dans l'espoir de réaliser un blocage qui s'opposerait à la force d'expulsion des clous. Le résultat n'a pas été satisfaisant: certes, l'expulsion des clous a diminué mais ex prix de nombreuses écailles génératrices d'instabilité rotatoire et parfois de fractures supracondyliennes. Lorsque le remplissage était bien réalisé, la protrusion des clous dans l'articulation était redoutée.

Une seconde tentative de remédier à ces mouvements a été la fixation des clous à l'aide de ciment acrylique pour sceller la fenêtre d'introduction des clous. Cette méthode de fixation rigide est sujette à beaucoup de critiques et n'a pas été suivie.

L'invention vise à améliorer, par l'emploi d'un clou perfectionné, les techniques actuellement connues, de manière à:

— créer un butoir qui permette une descente limitée des clous
— solidariser l'implant de l'os dans le plan horizontal pour contrôler les rotations.

Un implant perfectionné conforme à l'invention, pour le traitement des fractures trochantériennes, comprend un clou selon la forme de clou d'ENDER, comportant une première partie constituée d'une tige précourbée, ainsi qu, une seconde partie plate, appelée palette, an prolongement de la tige et dans laquelle est pratiquée une coulisse longitudinale et il est caractérisé en ce que, ladite coulisse longitudinale ayant une longueur voisine de 15 mm et une largeur voisine de 5 mm, une vis autotaraudeuse de diamètre inférieur à la largeur de la coulisse est passée dans ladite coulisse suivant une direction sensiblement perpendiculaire au plan de la palette. Après insertion à la partie inférieure de la coulisse et vissage dans l'apophyse inférieure, la vis autotaraudeuse limite la descente du clou par blocage sur la partie supérieure de la coulisse.

L'expérience a montré que les dimensions de la palette doivent être de préférence: longueur voisine de 20 mm et largeur voisine de 10 mm; 22 mm et 9 mm paraissant les dimensions optima.

La technique d'emploi est, dans ses phases initiales, dérivée de celle d'ENDER: réduction en léger valgus de 10° en moyenne, pratique d'une tranchée d'introduction des clous, passage des clous dans le canal médullaire.

La technique de fixation qui intervient alors est particulière à l'emploi des clous de l'invention. Elle consiste d'abord:

a) à s'assurer que le membre inférieure n'est pas en rotation externe. Il convient de le remettre en légère rotation interne dans le but d'éviter une fixation en mauvaise position.

b) à relâcher complètement la traction et impacter le foyer de la fracture en poussant le fémur de bas en haut.

Une fois a) et b) réalisés, commence la fixation proprement dite par la mise en place dans les coulisses d'une seule vis autotaraudeuse ou simple. Cette vis doit prendre au minimum deux clous, au maximum trois clous, en superposant les palettes l'une sur l'autre. Cette superposition est facilitée et est maintenue par une pince plate le temps de la mise en place de la vis dans la partie inférieure de la coulisse. La vis doit s'appuyer sur la conticale interne et prendre la corticale externe. Elle ne doit être serrée que légèrement. La direction de la vis doit être perpendiculaire au plan de la palette. Dans cette position, la force d'arrachement de la vis est nulle. Sa résistance est optimale.

De cette façon, l'ancrage antirotatoire est assuré d'emblée entre les clous et l'os. La rotation de l'un induit la rotation de l'autre, et le fragment céphalique suit le mouvement par l'intermédiaire des clous. Il faut signaler que dans la partie supérieure de la diaphyse l'élasticité des clous permet un certain degré de rotation (vrillage) incontrôlable par une fixation à distance.

Par ailleurs, la descente des clous est contrôlée par la vis qui joue la rôle de butoir d'arrêt. En effet, les clous peuvent glisser sur la vis de la distance que leur permet la coulisse. Cette distance est généralement suffisante pour que la fracture arrive à se stabiliser, d'autant plus que le foyer de fracture est impacté avant la mise en place la vis.

Un clou conforme à l'invention est décrit en détail ci-après par référence aux figures jointes.

La Fig. 1 est une vue partielle de face d'un clou pour implant conforme à l'invention.

La Fig. 2 est une vue en coupe d'un fémure avec fracture trochantérienne avec implant d'un faisceau de clous conforme à l'invention au moment de la mise en place de la vis.

La Fig. 3 est une vue partielle de profil de l'implant de la Fig. 2.

La Fig. 4 est une vue en coupe identique à la Fig. 2, mais après descente du faisceau de clous.

La Fig. 5 est une vue partielle de profil correspondant à la Fig. 4.

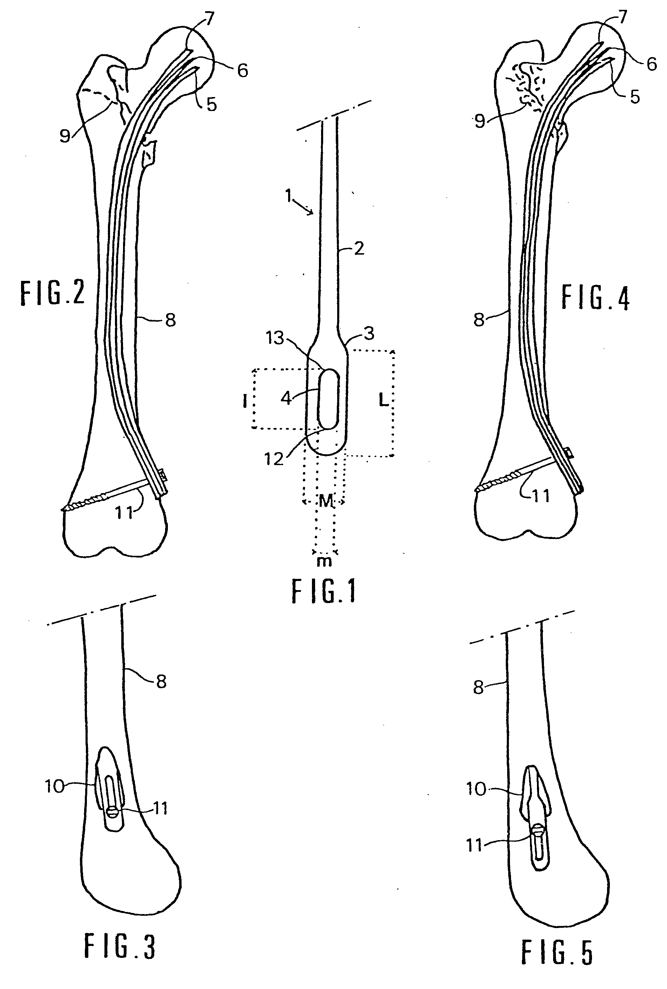

Un clou élastique à palette 1 conforme à l'invention est représenté partiellement à la Fig. 1. Il comporte une première partie 2 constituée d'une tige précourbée selon la forme du clou d'ENDER et une seconde partie plate 3, appelée palette, de longueur L et de largeur M, ces dimensions étant respectivement de préférence 22 mm et 9 mm. Dans la palette 3 est pratiquée une coulisse 4 de longueur 1 et de largeur m, ces dimensions étant de préférence respectivement 15 mm et 5 mm.

On a représenté aux Figs. 2 et 4 un faisceau de trois clous 5, 6 et 7, identiques au clou 1, implanté dans un fémur 8 présentant une fracture 9. Les clous ont été introduits par une tranchée d'introduction 10, visible sur les Figs. 3 et 5.

Dans les coulisses des trois clous est passée une vis autotaraudeuse 11, vissée, dans l'apophyse inférieure de fémur 8 et maintenant en superposition l'un sur l'autre les trois clous. La position de la vis 11, sur les Figs. 2 et 4, correspond au moment de sa mise en place, cette mise en place ayant lieu par passage de la vis dans la partie inférieure 12 des coulisses.

La descente du faisceau de clous est arrêtée au moment où la partie supérieure 13 des coulisses se bloque sur la vis 11. Cette situation est représentée aux Fig. 4 et 5.

## Revendication

Implant pour la réduction des fractures trochantériennes, comprenant un clou (1) à tige précourbée (2) avec palette (3) d'extrémité comportant une coulisse longitudinale (4), caractérisé en ce que, la coulisse longitudinale ayant une longueur voisine de 15 mm et une largeur voisine de 5 mm, une vis autotaraudeuse (11), orientée sensiblement perpendiculairement au plan de la palette (3) et d'un diamètre inférieure à la largeur de la coulisse (4) est passée dans ladite coulisse, d'où il ressort qu'après insertion à la partie inférieure (12) de la coulisse (4) et vissage dans l'apophyse inférieure, la vis auto-taraudeuse (11) limite la descente du clou (1) par blocage sur la partie supérieure (13) de la coulisse (4).

## Patentanspruch

Implant zur Reduktion von Trochanterbrüchen mit einem Nagel (1) mit vorgebogenem Schaft (2) und Endplatte (3), die ein Langloch (4) aufweist, dadurch gekennzeichnet, daß das Langloch (4) eine Länge von etwa 15 mm und eine Breite von etwa 5 mm aufweist, eine gewindeschneidende Schraube (11) mit einem Durchmesser kleiner als die Breite des Langloches (4) im wesentlichen senkrecht zur Ebene der Endplatte (3) durch das Langloch (4) geführt wird, wobei nach Einsetzen in das untere Apophyse die gewindeschneidende Schraube (11) die Abwärtsbewegung des Nagels (1) durch Anlage an dem oberen Teil (13) des Langloches (4) begrenzt.

## Claim

Implant for minimizing trochanter fractures, comprising a nail (1) having a prebent spindle (2) and an end blade (3) including a longitudinal slot (4), characterized in that, the longitudinal slot having a length about 15 mm and a width about 5 mm, a self-tapping screw (11) disposed substantially perpendicular to the plane of the blade (3) and having a diameter less than the width of the slot (4) is passed through said slot from which it emerges that, after inserting into the lower portion (12) of the slot (4) and screwing in the lower apophysis, the self-tapping screw (11) limits the descent of the nail (1) by locking on the upper position (13) of the slot (4).

FIG.2

FIG.1

FIG.4

FIG.3

FIG.5